# EUROPÄISCHE PATENTSCHRIFT

(11) **EP 1 884 191 B1**
(45) Veröffentlichungstag und Bekanntmachung des Hinweises auf die Patenterteilung: **23.09.2009**
(21) Anmeldenummer: 06016069.4
(22) Anmeldetag: 02.08.2006
(51) Int. Cl.: A61B 5/15

(54) **Blutentnahmesystem**
Lancet system
Système a piquer la peau

(43) Veröffentlichungstag der Anmeldung: 06.02.2008
(73) Patentinhaber: Roche Diagnostics GmbH, 68305 Mannheim (DE); F.HOFFMANN-LA ROCHE AG, 4070 Basel (CH)
(72) Erfinder: Winheim, Sven, 69181 Leimen (DE); Thoes, Bruno, 66287 Qierschied (DE); Döpper, Joachim, 64291 Darmstadt (DE)
(74) Vertreter: Petirsch, Markus

(56) Entgegenhaltungen:
- EP-A- 1 238 632
- EP-A- 1 625 825
- EP-A1- 0 565 970
- EP-A2- 1 090 584
- WO-A-92/03976
- WO-A-96/04857
- WO-A-20/06110573
- DE-A1- 3 925 940
- US-A- 5 645 555
- US-A- 5 797 940
- US-B1- 6 231 531

## Beschreibung

Die Erfindung betrifft ein Blutentnahmesystem zur Entnahme von Blut aus einem Körperteil für Diagnosezwecke.

Um Blut in geringen Mengen für Diagnosezwecke aus einem Körperteil, beispielsweise aus dem Finger oder dem Ohrläppchen, zu entnehmen, werden Lanzetten verwendet, die mit ihrer Spitze eine Wunde in das entsprechende Körperteil stechen. Dies geschieht manuell durch besonders trainiertes Personal oder durch besondere Blutentnahmesysteme, die sogenannte Stechgeräte und zugehörige Lanzetten einschließen.

In dem Gehäuse der Stechgeräte befindet sich ein Lanzettenantrieb zum Antreiben einer Stechbewegung der Lanzette, der bei manchen Geräten als Rotorantrieb ausgebildet ist, bei dem die Drehung eines Antriebsrotors in eine translatorische Bewegung der Lanzette bzw. eines Lanzettenhalters umgewandelt wird.

Solche Rotorantriebe sind schon lange bekannt. Beispielsweise wird in dem US-Patent 4,924,879 ein Pleuel für die Umsetzung der Rotation in eine Translationsbewegung verwendet. In dieser Ausführungsform werden durch den Pleuel auch Querkräfte auf die Lanzette übertragen, die als störend empfunden werden. Durch die Querkräfte wird zum einen die Führung der Lanzette negativ beeinflußt, zum anderen wird die im Körperteil entstehende Wunde vergrößert, was von dem Patienten als schmerzhaft empfunden wird.

Bei anderen Rotorantrieben ist in dem Antriebsrotor eine Ausnehmung vorgesehen, die als Steuerkurve dient und von einem Stift während der Rotation abgefahren wird. Diese Steuerkurvenkonstruktion ermöglicht ganz unterschiedliche Relationen zwischen der Drehbewegung und der Translationsbewegung, je nach Form der Steuerkurve. So kann beispielsweise auch während der Stechbewegung in Einstichrichtung eine andere Kurve abgefahren werden, als während der Rückführungsphase der Stechbewegung. Eine derartige Kurvensteuerung ist beispielsweise aus der EP 1034740 A1 bekannt, bei der ein um die Längsachse des Blutentnahmegeräts rotierender Rotor vorgesehen ist.

Die EP 1504718 A2 beschreibt ebenfalls ein Blutentnahmesystem mit einem Antriebsrotor, der um eine Achse rotiert, welche senkrecht zur Längsrichtung des Systems verläuft. Auch hier ist eine Steuerkurve von einer Ausnehmung gebildet, in die ein Steuerzapfen eingreift.

Den bekannten Blutentnahmegeräten ist gemeinsam, dass sie konstruktiv aufwendig und damit relativ teuer sind. Die Steuerzapfen müssen zum Teil aufgrund der Geometrie recht klein ausgebildet sein, so dass die Gefahr des Abbrechens besteht. Diesen bekannten Geräten ist weiter gemeinsam, dass die Antriebe zwangsgeführt sind, das heißt eine Kopplung zwischen dem Rotor und der Lanzette dergestalt ist, dass jeder Position des Rotors eindeutig eine Position der Lanzette entspricht.

Trotz umfangreichen Entwicklungsarbeiten auf diesem Gebiet und den dabei erzielten wesentlichen Verbesserungen besteht ein großes Interesse an einem Blutentnahmesystem, bei dem die Erzeugung der Wunde mit sehr geringen Schmerzen verbunden ist, das möglichst einfach zu bedienen, kompakt in der Bauform ist, trotzdem eine gewisse Robustheit aufweist und zudem kostengünstig herzustellen ist. Aufgabe der vorliegenden Erfindung ist es, ein derart verbessertes Blutentnahmegerät zu schaffen.

Die Aufgabe wird gelöst durch ein Blutentnahmesystem zur Entnahme von Blut aus einem Körperteil für Diagnosezwecke mit in den Merkmalen des Patentanspruchs 1.

Das Blutentnahmesystem umfaßt ein Gehäuse und einen Lanzettenantrieb zum Antreiben einer Stechbewegung einer Lanzette auf einem vorbestimmten Einstichweg. Der Lanzettenantrieb hat eine Antriebsfeder und einen Antriebsrotor. Der Antriebsrotor wird von der Antriebsfeder angetrieben und rotiert während der Stechbewegung um eine Achse. Zu dem Lanzettenantrieb gehört darüber hinaus ein Kopplungsmechanismus, durch den die Drehbewegung des Antriebsrotors in die Stechbewegung der Lanzette umgesetzt wird. In einer Vortriebsphase der Stechbewegung wird die Lanzette in Einstichrichtung bewegt, bis ihre Spitze in das Körperteil eindringt, um eine Wunde zu erzeugen. Der Kopplungsmechanismus bewegt die Lanzette auch in einer sich der Vortriebsphase anschließenden Rückführungsphase der Stechbewegung, bei der die Lanzette aus der Haut zurückgezogen wird.

Der Kopplungsmechanismus schließt ein mit der Lanzette gekoppeltes Translationselement ein, das zwei Führungswände mit jeweils einer Führungsfläche aufweist. Das Translationselement wird durch eine Führung auf einem translatorischen Bewegungsweg geführt. Die Kopplung zwischen Translationselement und Lanzette ist derartig ausgeführt, dass eine Bewegung des Translationselements auf dem translatorischen Bewegungsweg in eine erste Richtung eine Bewegung der Lanzette in der Vortriebsphase der Einstichrichtung bewirkt. Ebenso bewirkt die Bewegung des Translationselements in eine zweite Richtung eine Bewegung der Lanzette in der Rückführungsphase entgegen der Einstichrichtung. Die beiden Führungswände des Translationselements weisen jeweils eine Kontaktfläche auf, wobei eine Kontaktfläche in die erste Richtung des Translationselements orientiert ist und die andere Kontaktfläche in die zweite Richtung der Bewegung des Translationselements orientiert ist.

Die Orientierung der Kontaktfläche in eine Richtung der Bewegung des Translationselements ist so zu verstehen, dass die Flächennormale der Kontaktfläche eine Komponente in Richtung der Bewegung des Translationselements aufweist. Die Kontaktfläche verläuft somit quer zur Bewegungsrichtung des Translationselements. Bevorzugt steht sie senkrecht zu der Bewegungsrichtung, so dass die Flächennormale nur eine Komponente in Bewegungsrichtung des Translationselements aufweist.

Die Wirkverbindung zwischen der Bewegung des Translationselements und der Bewegung der Lanzette kann durch nahezu jede beliebige Kopplung geschehen. So kann das Translationselement entweder direkt mit der Lanzette gekoppelt sein. Die Kopplung kann beispielsweise einen Winkelhebel oder eine Umlenkung umfassen, um die Richtung der Bewegung des Translationselements in eine vorgegebene Richtung der Lanzettenbewegung zu übertragen.

Der Antriebsrotor des Blutentnahmesystems hat ein Steuerungselement mit einer um ein Zentrum des Steuerungselements umlaufenden, von dem Zentrum radial nach außen orientierten Steuerungsfläche. Während der Stechbewegung rotiert das Steuerungselement gemeinsam mit dem Antriebsrotor. Die Rotationsachse verläuft mit Abstand von dem Zentrum des Steuerungselements. Das Steuerungselement wird gemeinsam mit dem Antriebsrotor dabei von der Antriebsfeder angetrieben. Die Steuerungsfläche des Steuerungselements steht mit den Führungsflächen an den Führungswänden des Translationselements während der Rotation des Antriebsmotors derart in Kontakt, dass dadurch die Bewegung des Translationselements mindestens während eines Teil der Vortriebsphase und mindestens eines Teils der Rückführungsphase der Stechbewegung gesteuert wird.

Auf diese Weise läßt sich eine sehr direkte Umsetzung der Rotationsbewegung des Antriebsrotors in eine Translationsbewegung des Translationselements verwirklichen. Dies ermöglicht eine gute Führung, so dass nur kleine Wunden in der Haut des Patienten verursacht werden. Darüber hinaus läßt sich ein schnelles Einstechen in das Körperteil realisieren, was ebenso wie die kleinen Wunden zu einem verringerten Schmerzempfinden des Patienten führt. Diese Art des Kopplungsmechanismus hat den weiteren Vorteil, dass sehr wenige Bauteile verwendet werden. Das Blutentnahmesystem kann dadurch kostengünstig hergestellt werden. Auch kann auf eine aufwendige Mechanik verzichtet werden, wie sie beispielsweise bei der Verwendung von Steuerkurven notwendig ist. Hierdurch läßt sich insgesamt ein kostengünstiges Gerät schaffen, das eine kleine Bauform aufweist und darüber hinaus sehr robust ist.

Bei der umlaufenden Steuerfläche des Steuerungselements handelt es sich vorzugsweise um eine Mantelfläche in dem Sinn, dass sie von einer erzeugenden Geraden gebildet wird, die parallel zu der Rotationsachse des Antriebsrotors verläuft. Die Erzeugende muss jedoch nicht unbedingt eine Gerade sein. Wenn die Erzeugende gekrümmt ist, dann bevorzugt in der Weise, dass die entstehende Steuerungsfläche nach außen konvex gewölbt ist. Vorzugsweise sollte sie längs einer Linie ihres maximalen Abstands vom Zentrum des Steuerungselements parallel zur Rotationsachse verlaufen.

Der Begriff "rotieren" zur Beschreibung der Rotation des Steuerungselements wird hier allgemein dahingehend verstanden, dass er sowohl eine Drehung um eine eigene Achse (d.h. eine Achse, die durch das Steuerungselement hindurch, mithin innerhalb der umlaufenden Steuerungsfläche, verläuft), als auch eine Drehbewegung des Steuerungselements, vorzugsweise auf einer Kreisbahn, um eine Achse außerhalb der Steuerungsfläche beschreibt. Da die Drehung des Steuerungselements im vorliegenden Fall um eine Rotationsachse ausgeführt wird, die mit Abstand zum Zentrum des Steuerungselements verläuft, dreht sich das Steuerungselement jedenfalls um eine aus seinem Zentrum herausgerückte Rotationsachse. Das Steuerungselement führt also eine exzentrische Drehung durch, bei der sein eigenes Zentrum von der Rotationsachse verschieden ist.

Bevorzugt sind die Führungswände des Translationselementes derart ausgerichtet, dass die erste Kontaktfläche in Einstichrichtung orientiert ist, während die andere Kontaktfläche der zweiten Führungswand entgegen der Einstichrichtung orientiert ist. Hierdurch wird ein direktes Zusammenwirken zwischen dem Translationselement und der Lanzette erreicht. Die Bewegungsrichtung des Translationselements entspricht dann der Bewegungsrichtung der Lanzette. Die Kontaktfläche ist jetzt in bzw. entgegen der Einstichrichtung orientiert. Die Flächennormale der Kontaktfläche weist also eine Komponente auf, die in Richtung bzw. entgegen der Einstichrichtung verläuft. Bevorzugt ist die Fläche senkrecht zur Einstichrichtung ausgerichtet.

Da die Steuerungsfläche des Steuerungselements mit den Führungsflächen des Translationselements in Kontakt steht und so die Bewegung des Translationselements mindestens teilweise während der Vortriebsphase und der Rücktriebsphase steuert, ist zumindest eine teilweise Kopplung realisiert. Die Lanzette wird also von dem Steuerungselement über den Kopplungsmechanismus sowohl in Teilen der Vortriebsphase als auch der Rückführungsphase direkt geführt. Diese teilweise Kopplung kann bevorzugt auch auf die gesamte Stechbewegung ausgedehnt werden. In diesem Fall ist die Lanzette durch den Antriebsrotor zwangsgeführt. Es entsteht eine direkte Kopplung. Jede Stellung des Steuerungselements entspricht einer Stellung des Translationselements und damit letztlich der Lanzette. Man kann hier von einer synchronen Kopplung sprechen, da die Position des Steuerungselements einer eindeutigen Position der Lanzette zugeordnet werden kann.

Wie erwähnt kann das Steuerungselement des erfindungsgemäßen Blutentnahmesystems entweder so ausgebildet sein, dass seine Rotationsachse innerhalb des Steuerungselements oder alternativ auch außerhalb liegt. Ist die Rotationsachse innerhalb des Steuerungselements angeordnet, so kann das Steuerungselement bevorzugt durch einen Rand des Antriebsrotors gebildet werden. Eine Ausführungsform, bei der das Steuerungselement Teil des Antriebsrotors ist, ist besonders einfach, da nur ein Bauteil verwendet werden muss.

Auch wenn die Rotationsachse außerhalb des Steuerungselements liegt und das Steuerungselement bei der Rotation des Antriebsrotors auf einer Umlaufbahn um die Rotationsachse umläuft, kann das Steuerungselement Bestandteil des Antriebsrotors sein, insbesondere von einem an dem Antriebsrotor angebrachten Stift gebildet werden. Damit wird auch deutlich, dass der Antriebsrotor nicht notwendigerweise scheiben- oder radförmig sein muss. Seine Geometrie ist nicht auf eine nockenförmige Ausbildung beschränkt. Der allgemein verstandene Begriff "Antriebsrotor" umfasst vielmehr auch Fälle, bei denen das stiftförmige Steuerungselement am Ende eines rotierenden Armes angebracht ist.

In einer bevorzugten Ausführungsform des Blutentnahmesystems sind wenigstens die Kontaktflächen des Translationselements aus gleitoptimierten Werkstoffen hergestellt, die mit dem Steuerungselement in Kontakt stehen. Besonders bevorzugt ist das gesamte Translationselement aus gleitoptimierten Werkstoffen hergestellt. Es ist auch möglich, dass die Kontaktflächen oder das gesamte Translationselement mit einem Überzug versehen ist, der gleitoptimierte Eigenschaften aufweist. Als gleitoptimierte Werkstoffe kommen insbesondere alle Materialien in Betracht, die mit PTFE versetzt sind, wie z.B. POM PTFE. Auf diese Weise wird die Reibung zwischen dem Steuerungselement und dem Translationselement verringert. Der Verschleiß wird deutlich reduziert. Insgesamt ergibt sich dadurch eine einfache Handhabung des Geräts, da die Umwandlung der Rotationsbewegung des Steuerungselements in die Translationsbewegung des Translationselements ohne wesentliche Reibungsverluste durchgeführt wird.

Bevorzugt wird die Führung des Translationselements während seiner translatorischen Bewegung durch Führungsschienen gebildet, die in dem Gehäuse des Blutentnahmesystems angeordnet sind. Alternativ oder zusätzlich kann auch eine Zylinderführung verwendet werden. Dies bietet sich besonders dann an, wenn das Blutentnahmesystem im wesentlichen die Form eines langgestreckten Zylinders hat, die als sogenannte "Pencilform" bezeichnet wird. Insbesondere kann das Gehäuse des Blutentnahmesystems als Führung für das Translationselement dienen. Es sind auch andere Arten der Führung denkbar, solange sie den Zweck erfüllen, einen möglichst geraden Einstich der Lanzette zu gewährleisten und möglicherweise auftretende Vibrationen zu vermeiden.

Die Erfindung wird nachfolgend anhand von in den Figuren dargestellten Ausführungsbeispielen näher erläutert. Die darin dargestellten Besonderheiten können einzeln oder in Kombination verwendet werden, um bevorzugte Ausgestaltungen der Erfindung zu schaffen. Es zeigen:
- Fig. 1: ein schematisches Schnittbild einer ersten Ausführungsform eines Blutentnahmesystems;
- Fig. 2: eine Prinzipdarstellung einer zweiten Ausführungsform eines Blutentnahmesystems im Querschnitt und
- Fig. 3: eine Detaildarstellung eines Kopplungsmechanismus des Blutentnahmesystems nach Figur 2.

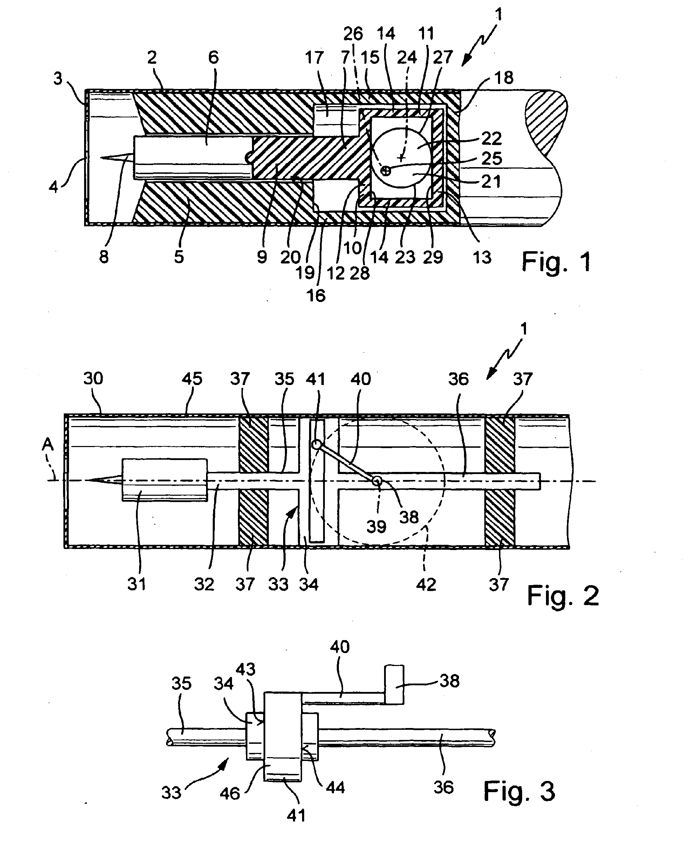

Das in Figur 1 in Form einer schematischen Prinzipdarstellung gezeigte Blutentnahmesystem 1 hat ein langgestrecktes Gehäuse 2, an dessen Vorderende 3 eine Öffnung 4 vorgesehen ist. Im Inneren des Gehäuses 2 ist eine Lanzettenführung 5 vorhanden, die eine Lanzette 6 auf ihren Einstichweg führt. Selbstverständlich kann die Lanzette 6 auf ihrem Einstichweg auch von dem Gehäuse 2 selbst geführt werden. Das Gehäuse 2 bzw. dessen Innenwand ist dann die Lanzettenführung 5.

Die Lanzette 6 ist mit einem Kopplungsmechanismus 7 gekoppelt und wird von ihm so gehalten, dass sich die Spitze 8 der Lanzette 6 auch dann in der gleichen Position befindet, wenn jeweils eine neue Lanzette 6 mit dem Kopplungsmechanismus 7 gekoppelt wird. Der Kopplungsmechanismus 7 umfaßt einen Lanzettenhalter 9, der an die Lanzette 6 angepaßt ist. An den Lanzettenhalter 9 des Kopplungsmechanismus 7 schließt sich ein Translationselement 10 an, das die Form eines Käfigs 11 aufweist. Der Käfig 11 hat eine vordere Führungswand 12 und eine hintere Führungswand 13 sowie zwei Seitenwände 14.

Das Translationselement 10 des Kopplungsmechanismus 7 wird von einer mit dem Gehäuse 2 verbunden Führung 15 auf einem geradlinigen Bewegungsweg geführt. Die Führung 15 ist im dargestellten Beispiel als Zylinderführung 16 ausgebildet und führt das Translationselement 10 während der Stechbewegung der Lanzette 6. Die Führung 15 kann durch das Gehäuse 2 gebildet werden. Insbesondere bei zylindrisch ausgebildeten Gehäusen 2 ist die Zylinderführung durch die Innenwand des Gehäuses bereits gegeben. Selbstverständlich kann die Führung 15 wie auch die Lanzettenführung 5 von dem gleichen Bauteil realisiert werden, z.B. kann die Lanzettenführung 5 gleichzeitig die Führung 15 sein. Insbesondere wenn das Gehäuse 2 die Lanzettenführung 5 bildet, kann es gleichzeitig als Führung für das Translationselement 10 dienen.

Die in Figur 1 dargestellte Zylinderführung 16 bildet im Inneren einen Führungsraum 17, der von einer hinteren Rückwand 18 begrenzt wird. In Richtung auf das Vorderende 3 wird der Führungsraum 17 von einer Vorderwand 19 abgeschlossen, die eine Bohrung 20 aufweist, durch welche der mit dem Translationselement 10 einstückig verbundene Lanzettenhalter 9 des Kopplungsmechanismus 7 hindurchragt. Die Rückwand 18 und die Vorderwand 19 des Führungsraum 17 weisen einen derart großen Abstand voneinander auf, dass sie den Bewegungsweg des Translationselements 10 nicht beschränken.

Innerhalb des Käfigs 11 des Translationselements 10 ist ein Antriebsrotor 21 angeordnet. Der Antriebsrotor 21 gehört zu einem Lanzettenantrieb, der auch hier nicht dargestellte Teile, insbesondere eine Antriebsfeder sowie eine Spannvorrichtung zum Spannen der Antriebsfeder umfaßt. Während der Stechbewegung der Lanzette 6 wird der Antriebsrotor 21 von der Antriebsfeder angetrieben und rotiert um eine Achse 26 herum.

Der radial nach außen orientierte Rand 23 des Antriebsrotors 21 bildet bei der hier dargestellten Ausgestaltung eine Steuerungsfläche 27. In der Ausführungsform nach Figur 1 bildet also der Antriebsrotor 21 zugleich das Steuerungselement 22. Sein Zentrum 24, das dem Zentrum der Steuerungsfläche 27 entspricht, ist von der Rotationsachse 26 beabstandet. Während der Rotation umläuft das Zentrum 24 des Steuerungselements 22 die Rotationsachse 26 auf einer kreisförmigen Umlaufbahn.

Das Steuerungselement 22 hat eine von seinem Zentrum 24 radial nach außen orientierte Steuerungsfläche 27. Die Steuerungsfläche 27 ist derart gebildet, dass längs einer um das Zentrum 24 umlaufenden Linie die Flächennormalen radial vom Zentrum 24 nach außen gerichtet sind.

Die Steuerungsfläche 27 des Steuerungselements 22 steht in Kontakt mit einer Kontaktfläche 28 der vorderen Führungswand 12 des Translationselements 10 und mit einer Kontaktfläche 29 der hinteren Führungswand 13 des Translationselements 10. Die Kontaktfläche 28 ist entgegen der Einstichrichtung der Lanzette 6 orientiert; die Kontaktfläche 29 in Einstichrichtung.

In Figur 1 ist eine permanente Kopplung des Steuerungselements 22 mit dem Translationselement 10 gezeigt, bei der das Steuerungselement 22 gleichzeitig (selbstverständlich mit einem kleinen, eine reibungsarme Rotation ermöglichenden Spiel) an beiden Kontaktflächen 28 und 29 anliegt. Derartige Kopplungen werden auch als Zwangskopplung bezeichnet. Die Lanzette 6 ist dabei zwangsgeführt, das heisst jeder Position des rotierenden Steuerungselements 22 kann eine eindeutige Position des Translationselements 10 bzw. der Lanzette 6 zugeordnet werden.

In Figur 1 ist die Stellung des Steuerungselementes 22 im Umkehrpunkt der Stechbewegung gezeigt. Während der anschließenden Rückführungsphase der Stechbewegung übt das Steuerungselement 22 eine Kraft auf die Kontaktfläche 29 aus und drückt das Translationselement 10 entgegen der Einstichrichtung der Lanzette 6. Während der Vortriebsphase wirkt das Steuerungselement 22 mit einer Kraft auf die Kontaktfläche 28 und bewegt das Translationselement 10 in Einstichrichtung. Somit wird auf einfache Weise die Rotationsbewegung des Steuerungselements 22 in eine translatorische Bewegung des Translationselements 10 umgesetzt, wobei während der Drehung des Steuerungselements 22 um die Rotationsachse 26 eine Kraft der Steuerungsfläche 27 auf jeweils wenigstens eine der Kontaktflächen 28,29 wirkt.

Bevorzugt laufen die Kontaktflächen 28 und 29 des Translationselements 10 in einer zu der Rotationsachse 26 senkrechten Ebene gerade. Dadurch werden quer zu der Einstichrichtung wirkende Kräfte vermieden.

Es treten keine Querkräfte auf, so dass die gesamte von dem Steuerungselement 22 ausgeübte Kraft in bzw. entgegen der Einstichrichtung gerichtet ist.

Besonders bevorzugt ist eine Ausführungsform, bei der wenigstens die Kontaktfläche 28 des Translationselements 10 im wesentlichen senkrecht zur Einstichrichtung der Lanzette 6 ausgerichtet ist. Vorteilhaft sind beide Kontaktflächen 28 und 29 senkrecht zur Lanzette 6 ausgerichtet. Dadurch wird die Reibung zwischen dem Steuerungselement 22 und dem Translationselement 10 minimiert und ein Verkanten des Translationselements 10 auf seinem translatorischen Weg in Einstichrichtung und entgegen der Einstichrichtung vermieden. Unterstützt wird die gute Kraftübertragung durch die Führung 15, so dass insgesamt ein vibrationsfreier Einstich der Lanzette 6 in das Körperteil erzielt wird.

Alternativ zu dem in Figur 1 gezeigten Translationselement 10 kann der Abstand zwischen der vorderen Führungswand 12 und der hinteren Führungswand 13 größer sein als der Durchmesser des Steuerungselements 22. Der Abstand der beiden Führungswände 12 und 13 wird unter Berücksichtigung des Durchmessers und der Exzentrizität des Rotors so gewählt, dass das Steuerungselement 22 während mindestens der Hälfte der Stechbewegung mit einer der beiden Kontaktflächen 28, 29 des Translationselements 10 so in Kontakt steht, dass jeder Stellung des Steuerungselements 22 eine Stellung des Translationselements 10 und damit der Lanzette 6 entspricht. Während der Vortriebsphase wird die Kraft des Steuerungselements 22 mindestens zeitweise auf die Kontaktfläche 28 übertragen; während der Rückführungsphase wird die von dem Steuerungselement 22 ausgeübte Kraft wenigstens zeitweise auf die Kontaktfläche 29 übertragen. Es liegt in dem Fall keine dauerhaft zwangsgeführte Kopplung zwischen dem Steuerungselement 22 und dem Translationselement 10 vor.

Alternativ zu dem als Käfig 11 ausgeführten Translationselement 10 ist auch ein Translationselement 10 möglich, das eine vordere Führungswand 12 und eine hintere Führungswand 13 ohne Verbindungswände umfasst. Diese Führungswände 12, 13 können beispielsweise als Stege ausgebildet sein, die von einem Grundkörper des Translationselements hervorstehen. Das Steuerungselements 22 muss nicht umschlossen sein. Lediglich auf einem Teil der Vortriebsphase und einem Teil der Rückführungsphase muss der Kontakt zwischen dem Steuerungselement 22 und der vorderen Führungswand 12 bzw. der hinteren Führungswand 13 gewährleistet sein, damit mindestens eine zeitweise Kopplung zwischen dem Steuerungselement 22 und dem Translationselement 10 realisiert ist.

Die Figur 2 zeigt eine alternative Ausführungsform eines Blutentnahmesystems 1, das ein Gehäuse 30 mit einer Lanzettenführung und einem nicht dargestellten Lanzettenantrieb hat. Eine Lanzette 31 ist mit einem Kopplungsmechanismus 32 verbunden, der ein Translationselement 33 umfaßt, das einen Käfig 34 mit Führungswänden 34a und 34b und zwei sich gegenüberliegende Führungsstangen 35,36 aufweist. Die Führungsstangen 35,36 erstrecken sich längs einer Gehäusemittelachse A, wobei die Führungsstange 35 zwischen dem Käfig 34 und der Lanzette 31 angeordnet ist; die Führungsstange 36 erstreckt sich vom Käfig 34 aus von der Lanzette 31 weg. Die Stege 35,36 werden von einer Führung in Einstichrichtung geführt, die aus Führungselementen 37 besteht.

Ebenfalls auf der Gehäusemittelachse A ist ein Antriebsrotor 38 angeordnet, der während der Stechbewegung der Lanzette 31 von einer nicht dargestellten Antriebsfeder des Lanzettenantriebs gedreht wird. Der Antriebsrotor 38 rotiert um eine Rotationsachse 39. An dem Antriebsrotor 38 ist ein Rotorarm 40 befestigt, der, wie hier, einstückig mit dem Antriebsrotor 38 sein kann. Am fernen Ende des Rotorarms 40 ist ein fest gekoppeltes Steuerungselement 41 vorgesehen, das von dem Käfig 34 umschlossen wird.

In dem in Figur 2 dargestellten Ausführungsbeispiel liegt die Rotationsachse 39 außerhalb des Steuerungselements 41 und das Steuerungselement 41 läuft bei der Rotation des Antriebsrotors 38 auf einer gestrichelt dargestellten Umlaufbahn 42, die einen Kreis um die Rotationsachse 39 herum beschreibt.

Das Steuerungselement 41 ist während seiner Rotationsbewegung um die Rotationsachse 39 stets mit dem Käfig 34 gekoppelt; es liegt also an den Kontaktflächen 43,44 des Käfigs 34 so an, dass eine direkte Kopplung zwischen dem Antriebsrotor 38 und dem Translationselement 33 und somit letztlich mit der Lanzette 31 besteht.

Der Käfig 34 selbst wird ebenfalls im Gehäuse 30 geführt, wobei die Innenwände des Gehäuses 30 als Führungsschienen 45 ausgebildet sind. Insbesondere durch die Ausführung des Translationselements 33 als Käfig 34 mit senkrecht zur Einstichrichtung ausgerichteten Kontaktflächen 43,44 werden Querkräfte minimiert. Die Kraftübertragung in Einstichrichtung und entgegen der Einstichrichtung ist optimiert.

Figur 3 zeigt einen Längsschnitt entlang der Gehäusemittelachse A aus Figur 2. Der nur aus einer Lagerwelle bestehende Antriebsrotor 38 ist mit dem Rotorarm 40 fest verbunden. Am nicht gezeigten Ende des wellenförmigen Antriebsrotors 38 ist eine nicht dargestellte Antriebsmechanik angeordnet. Der Rotorarm 40 ist an seinem anderen Ende mit dem Steuerungselement 41 verbunden, das bevorzugt als Stift 46 ausgebildet ist. Durch die starre Kopplung über den Rotorarm 40 besteht eine feste Verbindung zwischen dem Stift 46 und dem Antriebsrotor 38. Der Stift 46 überträgt die von dem Antriebsrotor 38 ausgeübten Kräfte auf die Kontaktflächen 43 und 44 des Käfigs 34 des Translationselements 33.

Deutlich zu erkennen ist in Figur 3, dass der Stift 46 nicht aus dem Käfig 34 herausrutschen kann, da er den Käfig 34 überragt und das Spiel zwischen dem Stift 46 und den geraden Kontaktflächen 43,44 des Käfigs 34 gering ist. Insbesondere wird ein Herausrutschen des Stifts 46 dann vermieden, wenn die Führung so ausgebildet ist, dass das Translationselement 33 ausschließlich eine translatorische Bewegung in und entgegen der Einstichrichtung der Lanzette 31 durchführen kann und eine Bewegung in die beiden anderen Raumrichtungen, insbesondere ein Verkanten, ausgeschlossen ist.

Die Kopplung von Translationselement und Lanzette ist derart gegeben, dass eine Bewegung des Translationselementes in eine erste Richtung eine Bewegung der Lanzette in der Vortriebsphase in Einstichrichtung bewirkt. Eine Bewegung des Translationselementes in eine zweite Richtung verursacht eine Bewegung der Lanzette in der Rückführungsphase entgegen der Einstichrichtung. Die Führungsflächen sind derart ausgerichtet, dass die eine Führungsfläche in die erste Richtung der Bewegung des Translationselementes orientiert ist und die andere Führungsfläche in die zweite Richtung der Bewegung des Translationselementes orientiert ist.

## Patentansprüche

1. Blutentnahmesystem zur Entnahme von Blut aus einem Körperteil für Diagnosezwecke, umfassend
ein Gehäuse (2) und
einen Lanzettenantrieb zum Antreiben einer Stechbewegung einer Lanzette (6) auf einem vorbestimmten Einstichweg, mit
einer Antriebsfeder,
einem von der Antriebsfeder angetriebenen, während der Stechbewegung um eine Achse rotierenden Antriebsrotor (21) und
einem Kopplungsmechanismus (7), durch den die Drehbewegung des Antriebsrotors (21) in die Stechbewegung umgesetzt wird, wobei die Lanzette (6) in einer Vortriebsphase der Stechbewegung in Einstichrichtung bewegt wird, bis ihre Spitze (8) in das Körperteil eindringt, um eine Wunde zu erzeugen und in einer sich der Vortriebsphase anschließenden Rückführungsphase der Stechbewegung aus der Haut zurückgezogen wird,
wobei
der Kopplungsmechanismus (7) ein mit der Lanzette (6) gekoppeltes durch eine Führung (15) auf einem translatorischen Bewegungsweg geführtes Translationselement (10) einschließt,
das Translationselement (10) mit der Lanzette (6) derartig gekoppelt ist, dass durch eine Bewegung des Translationselementes (10) in Einstichrichtung eine Bewegung der Lanzette (6) in der Vortriebsphase in Einstichrichtung bewirkt wird und
durch eine Bewegung des Translationselementes (10) entgegen der Einstichrichtung eine Bewegung der Lanzette (6) in der Rückführungsphase entgegen der Einstichrichtung bewirkt wird,
der Antriebsrotor (21) ein Steuerungselement (22) mit einer um ein Zentrum (24) des Steuerungselementes (22,41) umlaufenden von dem Zentrum (24) radial nach außen orientierten Steuerungsfläche (27) aufweist,
**dadurch gekennzeichnet, dass**
das Transtationsetement (10) zwei Führungswände (12,13) mit jeweils einer Kontaktfläche (28,29) aufweiset, wobei die eine Kontaktfläche (29) in Einstichrichtung und die andere Kontaktfläche (28) entgegen der Einstichrichtung orientiert sind und
wobei
das Steuerungselement (22) während der Stechbewegung, mit dem Antriebsrotor um eine innerhalb der umlaufenden Steuerungsfläche (27) des Steuerungselements (22) verlaufenden Rotationsachse (26) rotiert, die mit Abstand von dem Zentrum (24) des Steuerungselements (22) verläuft, und
die Kontaktfläche (28,29) des Translationselements (10) senkrecht zur Einstichrichtung ausgerichtet sind,
die Steuerungsfläche (27) des Steuerungselementes (22) während der Rotation des Antriebsrotors (21) derartig mit den Kontaktflächen (28,29) an den Führungswänden (12,13) des Translationselements (10,33) in Kontakt steht, dass dadurch die Bewegung des Translationselementes (10) mindestens während eines Teils der Vortriebsphase und mindestens während eines Teils der Rückführungsphase der Stechbewegung gesteuert wird und dass quer zur Einstichrichtung wirkende Kräfte vermieden werden.

2. Blutentnahmesystem nach Anspruch 1, **dadurch gekennzeichnet dass** die gesamte von dem Steuerungselement (22) ausgeübte Kraft in bzw. entgegen der Einstichrichtung gerichtet ist.

3. Blutentnahmesystem nach einem der vorhergehenden Ansprüche, **dadurch gekennzeichnet, dass** das Steuerungselement (22) von einem Rand des Antriebsrotors (21) gebildet wird.

4. Blutentnahmesystem nach einem der vorhergehenden Ansprüche, **dadurch gekennzeichnet, dass** das Steuerungselement (22) während mindestens der Hälfte der Stechbewegung derart mit den beiden Kontaktflächen (28,29) des Translationselements (10) in Kontakt steht, dass jeder Stellung des Steuerungselements (22) eine Stellung des Translationselements (10) und damit der Lanzette (6) entspricht.

5. Blutentnahmesystem nach einem der vorhergehenden Ansprüche, **dadurch gekennzeichnet, dass** wenigstens die mit dem Steuerungselement (22) in Kontakt stehenden Kontaktflächen (28,29) des Translationselements (10), bevorzugt das gesamte Translationselement (10), aus gleitoptimierten Werkstoffen hergestellt sind.

6. Blutentnahmesystem nach einem der vorhergehenden Ansprüche, **dadurch gekennzeichnet, dass** die Führung (15) Führungsschienen oder einen Führungszylinder (16) aufweist.

7. Blutentnahmesystem nach einem der vorhergehenden Ansprüche, **dadurch gekennzeichnet, dass** das Translationselement (10) in Form eines Käfigs (11) ausgebildet ist, der das Steuerungselement (22) umschließt.

## Claims

1. Blood withdrawal system for producing blood from a body part for diagnostic purposes, comprising
a housing (2) and
a lancet drive for driving a puncturing movement of a lancet (6) on a predetermined puncturing path, the lancet drive having
a drive spring,
a drive rotor (21) which rotates about an axis during the puncturing movement and is driven by the drive spring, and
a coupling mechanism (7) by means of which the rotational movement of the drive rotor (21) is converted into the puncturing movement, wherein the lancet (6) is moved during a forward phase of the puncturing movement in the puncturing direction until its tip (8) penetrates into the body part to create a wound and is retracted from the skin in a retraction phase which follows the forward phase of the puncturing movement,
wherein
the coupling mechanism (7) includes a translation element (10) which is coupled to the lancet (6) and is guided by a guide (15) on a translational movement path,
the translation element (10) is coupled to the lancet (6) in such a manner that a movement of the lancet (6) during the forward phase in the puncturing direction is effected by a movement of the translation element (10) in a first direction and
a movement of the lancet (6) in the retraction phase against the puncturing direction is effected by a movement of the translation element (10) against the puncturing direction,
the drive rotor (21) has a control element (22) with a control surface (27) which runs around a center (24) of the control element (22, 41) and which is oriented radially outwardly from the center (24),
**characterized in that**
the translation element (10) has two guide walls (12, 13) each with a contact surface (28, 29), one contact surface (29) being oriented in the puncturing direction and another contact surface (28) being oriented against the puncturing direction and
wherein
during the puncturing movement, the control element (22) rotates with the drive rotor about an axis of rotation (26) which runs within the encompassing control surface (27) of the control element (22) and at a distance from the center (24) of the control element (22), and
the contact surfaces (28, 29) of the translation element (10) are perpendicular to the puncturing direction,
the control surface (27) of the control element (22) is in contact with the contact surfaces (28, 29) of the guide walls (12, 13) of the translation element (10, 33) during the rotation of the drive rotor (21) such that the movement of the translation element (10) is controlled thereby at least during a portion of the forward phase and at least during a portion of the retraction phase of the puncturing movement, and such that forces acting across to the puncturing direction are prevented.

2. Blood withdrawal system according to Claim 1, **characterized in that** the entire force exerted by the control element (22) is directed in or against the puncturing direction, respectively.

3. Blood withdrawal system according to any one of the preceding claims, **characterized in that** the control element (22) is formed by a circumferential wall of the drive rotor (21).

4. Blood withdrawal system according to any one of the preceding claims, **characterized in that** the control element (22) is during at least half of the puncturing movement in contact with the two contact surfaces (28, 29) of the translation element (10) in such a manner that each position of the control element (22) corresponds to the position of the translation element (10) and thus to the position of the lancet (6).

5. Blood withdrawal system according to any one of the preceding claims, **characterized in that** at least the contact surfaces (28, 29) of the translation element (10) in contact with the control element (22), preferably the entire translation element (10) are manufactured from friction-optimized materials.

6. Blood withdrawal system according to any one of the preceding claims, **characterized in that** the guide (15) comprises guide rails or a guide cylinder (16).

7. Blood withdrawal system according to any one of the preceding claims, **characterized in that** the translation element (10) is formed as a cage (11) surrounding the control element (22).

## Revendications

1. Système de prélèvement sanguin pour prélever du sang sur une partie du corps à des fins de diagnostic, comprenant
un boîtier (2), et
un entraînement de lancette pour entraîner un mouvement de piqûre d'une lancette (6) sur un trajet de piqûre prédéterminé, comportant
un ressort d'entraînement,
un rotor d'entraînement (21) entrainé par le ressort d'entraînement et tournant autour d'un axe pendant le mouvement de piqûre, et
un mécanisme de couplage (7) au moyen duquel le mouvement de rotation du rotor d'entraînement (21) est transformé en un mouvement de piqûre, la lancette (6), durant une phase d'avancement du mouvement de piqûre, se déplaçant dans le sens de la piqûre jusqu'à ce que sa pointe (8) pénètre dans la partie du corps pour provoquer une blessure, et se retirant de la peau durant une phase de recul du mouvement de piqûre subséquente à la phase d'avancement,
ledit mécanisme de couplage (7) incluant un élément de translation (10) couplé à la lancette (6) et dirigé par un guide (15) sur un trajet de mouvement translatoire,
ledit élément de translation (10) étant couplé à la lancette (6) de telle manière qu'un mouvement de l'élément de translation (10) dans le sens de la piqûre provoque un mouvement de la lancette (6) dans le sens de la piqûre durant la phase d'avancement et qu'un mouvement de l'élément de translation (10) en sens inverse de la piqûre provoque un mouvement de la lancette (6) en sens inverse de la piqûre durant la phase de recul,
ledit rotor d'entraînement (21) comprenant un élément de commande (22) muni d'une surface de commande (27) tournant autour d'un centre (24) de l'élément de commande (22, 41) et orientée radialement vers l'extérieur depuis ledit centre (24),
**caractérisé en ce que**
l'élément de translation (10) comprend deux parois de guidage (12, 13) munies chacune d'une surface de contact (28, 29), l'une (29) desdites surfaces de contact étant orientée dans le sens de la piqûre et l'autre surface de contact (28) en sens inverse de la piqûre, et
l'élément de commande (22), pendant le mouvement de piqûre, tournant avec le rotor d'entraînement autour d'un axe de rotation (26) passant à l'intérieur de la surface de commande (27) en rotation et s'étendant à distance du centre (24) de l'élément de commande (22), et
les surfaces de contact (28, 29) de l'élément de translation (10) étant orientées perpendiculairement au sens de la piqûre,
la surface de commande (27) de l'élément de commande (22), pendant la rotation du rotor d'entraînement (21), étant en contact avec les surfaces de contact (28, 29) sur les parois de guidage (12, 13), permettant ainsi de guider le mouvement de l'élément de translation (10) au moins pendant une partie de la phase d'avancement et au moins pendant une partie de la phase de recul et d'éviter des forces agissant perpendiculairement au sens de la piqûre.

2. Système de prélèvement sanguin selon la revendication 1, **caractérisé en ce que** toute la force exercée par l'élément de commande (22) est dirigée respectivement dans le sens et en sens inverse de la piqûre.

3. Système de prélèvement sanguin selon l'une des revendications précédentes, **caractérisé en ce que** l'élément de commande (22) est formé par un bord du rotor d'entraînement (21).

4. Système de prélèvement sanguin selon l'une des revendications précédentes, **caractérisé en ce que** l'élément de commande (22), pendant au moins la moitié du mouvement de piqûre, est en contact avec les deux surfaces de contact (28, 29) de l'élément de translation (10) de telle sorte qu'à chaque position de l'élément de commande (22) correspond une position de l'élément de translation (10) et donc de la lancette (6).

5. Système de prélèvement sanguin selon l'une des revendications précédentes, **caractérisé en ce qu'**au moins les surfaces de contact (28, 29) de l'élément de translation (10) en contact avec l'élément de commande (22), de préférence l'ensemble de l'élément de translation (10), sont fabriquées à partir de matériaux à glissement optimisé.

6. Système de prélèvement sanguin selon l'une des revendications précédentes, **caractérisé en ce que** le guide (15) comprend des rails de guidage ou un cylindre de guidage (16).

7. Système de prélèvement sanguin selon l'une des revendications précédentes, **caractérisé en ce que** l'élément de translation (10) est réalisé sous forme de cage (11) enveloppant l'élément de commande (22).
